# FASCICULE DE BREVET EUROPEEN

(11) **EP 2 254 584 B1**
(45) Date de publication et mention de la délivrance du brevet: **13.07.2016**
(21) Numéro de dépôt: 09707189.8
(22) Date de dépôt: 22.01.2009
(51) Int. Cl.: A61L 27/52, A61K 33/30, A61K 31/738, A61K 31/728, A61K 8/73, A61K 8/91, A61K 8/27, A61P 1/02, A61P 25/00, A61P 19/02, A61P 17/02, A61Q 19/08, A61L 27/54, A61K 31/315

(54) **PRODUITS INJECTABLES BIOCOMPATIBLES A LIBERATION DE ZINC ET/OU DE SEL DE SACCHARIDE SOUS FORME DE ZINC ET LEURS UTILISATIONS**
BIOKOMPATIBLE INJEKTIONSPRÄPARATE MIT FREISETZUNG VON ZINK UND/ODER EINEM ZINK-FORM-SACCHARIDSALZ UND IHRE VERWENDUNG
BIOCOMPATIBLE INJECTABLE PRODUCTS WITH ZINC AND/OR ZINC-FORM SACCHARIDE-SALT RELEASE, AND USES THEREOF

(30) Priorité: 07.02.2008 FR 0850790
(43) Date de publication de la demande: 01.12.2010
(73) Titulaire: Keysan Consulting, 13080 Luynes (FR)
(72) Inventeur: HERMITTE, Laurence, F-13080 Luynes (FR)
(74) Mandataire: Regimbeau
(86) Numéro de dépôt international: PCT/EP2009/050726
(87) Numéro de publication internationale: WO 2009/098127

(56) Documents cités:
- CN-A- 1 973 828
- FR-A- 2 865 737
- FR-A- 2 900 575
- US-B1- 6 608 043
- DATABASE MEDLINE [Online] US NATIONAL LIBRARY OF MEDICINE (NLM), BETHESDA, MD, US; juin 2006 (2006-06), NANOBASHVILI K V ET AL: "[Cytological issues of Curiosin's influence on the inflammatory processes in parodontal tissues]" XP002494973 Database accession no. NLM16905810 & GEORGIAN MEDICAL NEWS JUN 2006, no. 135, juin 2006 (2006-06), pages 57-60, ISSN: 1512-0112
- OLIVEIRA ET AL: "Intratesticular injection of a zinc-based solution as a contraceptive for dogs" THERIOGENOLOGY, LOS ALTOS, CA, US, vol. 68, no. 2, 13 juin 2007 (2007-06-13), pages 137-145, XP022114405 ISSN: 0093-691X
- ISHIHARA T ET AL: "Role of zinc in formulation of PLGA/PLA nanoparticles encapsulating betamethasone phosphate and its release profile" JOURNAL OF CONTROLLED RELEASE, ELSEVIER, AMSTERDAM, NL, vol. 105, no. 1-2, 20 juin 2005 (2005-06-20), pages 68-76, XP004933082 ISSN: 0168-3659

## Description

La présente invention se rapporte au domaine des produits injectables biocompatibles à visée cosmétique et médicale, notamment chirurgicale. Elle concerne tout particulièrement un produit injectable biocompatible à libération de zinc et/ou d'au moins un sel de saccharide sous forme de zinc.

Depuis plusieurs années, on constate un intérêt croissant pour les produits permettant le comblement, le remplacement, l'augmentation de volume d'un tissu biologique et/ou le remplacement, la supplémentation d'un fluide biologique, aussi bien dans le domaine cosmétique que médical.

Ainsi, par exemple l'utilisation de produit permettant l'augmentation de volume d'un tissu biologique est particulièrement intéressante dans le domaine cosmétique, pour traiter les rides et ridules mais aussi pour redéfinir les contours du visage, du corps. Par exemple, dans le cas de malades atteints de prognathie mandibulaire, chez lesquels on observe un aplatissement de la lèvre supérieure et un développement exagéré de la lèvre inférieure, les produits permettant d'augmenter le volume de la lèvre supérieure sont, esthétiquement particulièrement profitables.

Les produits d'augmentation de volume, de comblement, de remplacement d'un tissu biologique, ou de remplacement, de supplémentation d'un fluide biologique peuvent également être utiles dans le domaine médical.

Par exemple, on connaît dans l'art antérieur, l'utilisation de produits destinés à supplémenter le liquide synovial chez des patients atteints de dégénérescence articulaire.

Des produits pour le traitement des maladies arthritiques comprenant en tant que principe actif un complexe d'acide hyaluronique et de zinc sont décrits dans le brevet américain US6608043. Ce complexe est obtenu en mélangeant une solution aqueuse de hyaluronate de sodium avec une solution aqueuse de sel de zinc. Cependant, ce produit ne possède pas d'effet anti-inflammatoire, anti-infectieux et cicatrisant plus ou moins prolongé, ni une libération de zinc plus ou moins prolongé.

Des produits ayant un effet anti-inflammatoire sur des tissus parodontaux comprenant l'acide hyaluronique dans lequel du zinc est dispersé, sont décrits par Nanobashvill (Nanobashvill et al. « Cytological Issues of Curiosin's influence on the inflammatory processes in parodontal tissues", Medecine (NLM), Bethesda, MD, US, juin 2006). Cependant, ces produits ne sont pas injectables et ne permettent pas de moduler la libération du zinc et des sels de polysaccharides.

On connaît également dans l'art antérieur des produits dits « viscoélastiques » qui sont employés lors de chirurgies ophtalmiques afin de remplacer ou supplémenter l'humeur aqueuse (lors de la chirurgie de la cataracte ou dans le cadre de chirurgie réfractive) ou l'humeur vitrée (dans le cas d'une vitrectomie), tel que décrit dans le Brevet Américain US 4716154.

En outre, de tels produits peuvent parfois être avantageux par rapport aux chirurgies classiques, par exemple dans des maladies dégénératives du disque intervertébral pour remplacer le nucleus pulposus.

Un certain nombre de produits injectables biocompatibles a permis de combler, remplacer, augmenter le volume d'un tissu biologique et/ou remplacer, supplémenter un fluide biologique. Toutefois, certains d'entre eux ne peuvent être utilisés efficacement ni dans le domaine cosmétique ni dans le domaine médical du fait de leurs effets secondaires indésirables et/ou de leur faible efficacité.

Ainsi, certains de ces produits ne sont pas adaptés à un usage cosmétique ou médical du fait des douleurs, oedèmes, rougeurs temporaires qu'ils provoquent au niveau du site d'injection. Ces effets secondaires indésirables sont caractéristiques de la réponse inflammatoire induite par l'injection de ces produits.

De plus, l'efficacité de certains de ces produits, pourrait être améliorée en accélérant la cicatrisation consécutive à leur injection.

Il est donc intéressant de développer des produits injectables biocompatibles permettant de combler, remplacer, augmenter le volume d'un tissu biologique et/ou remplacer, supplémenter un liquide biologique, tout en ayant une activité cicatrisante et en permettant d'atténuer les effets de la réponse inflammatoire.

Les inventeurs ont maintenant établi un produit permettant de résoudre en tout ou partie les problèmes évoqués ci-dessus.

Les inventeurs ont mis au point un produit injectable biocompatible permettant notamment de combler, remplacer, augmenter le volume d'un tissu biologique et/ou remplacer, supplémenter un liquide biologique, tout en ayant une activité cicatrisante et anti-inflammatoire.

En effet, les inventeurs ont développé des produits injectables biocompatibles qui permettent de moduler la cinétique de libération du zinc et/ou d'au moins un sel de saccharide sous forme de zinc, à partir d'une matrice.

Les produits selon l'invention permettent, notamment, de libérer très tôt après l'administration du produit, du zinc et/ou au moins un sel de saccharide sous forme de zinc, ayant un effet anti-inflammatoire, cicatrisant et anti-infectieux. Les produits selon l'invention permettent ainsi de réduire les effets secondaires indésirables de la réponse inflammatoire associée à l'administration du produit.

En outre, en réduisant la réponse inflammatoire, cette libération permet d'augmenter la rémanence du produit, en ralentissant sa cinétique de dégradation.

De plus, en modulant la cinétique de libération du zinc et/ou d'au moins un sel de saccharide sous forme de zinc, les produits selon l'invention permettent d'obtenir un effet anti-inflammatoire, anti-infectieux et cicatrisant plus ou moins prolongé ainsi qu'une une rémanence *in vivo* du produit plus ou moins longue, en fonction de l'application visée.

La présente description se rapporte à un produit injectable biocompatible à libération de zinc et/ou d'au moins un sel de saccharide sous forme de zinc, comprenant :
- une matrice comprenant au moins un polymère biocompatible ; et
- du zinc et/ou au moins un sel de saccharide sous forme de zinc ;
dans lequel ledit zinc et/ou ledit au moins un sel de saccharide sous forme de zinc sont dispersés, réticulés et/ou greffés dans la matrice ; et
dans lequel la teneur en zinc, sous forme de zinc dispersé dans la matrice et/ou sous forme d'au moins un sel de saccharide sous forme de zinc dispersé dans la matrice, est compris entre 0,01 et 10 %, de préférence entre 0,05 et 5 % et tout préférentiellement entre 0,1 et 3 % en poids par rapport au poids total de la matrice.

Selon un premier aspect, la première invention concerne un produit tel que défini dans la revendication 1.

La présence de zinc, sous forme de zinc dispersé dans la matrice et/ou sous forme d'au moins un sel de saccharide sous forme de zinc dispersé dans la matrice, à une teneur comprise entre 0,01 et 10 % en poids par rapport au poids total de la matrice, permet d'obtenir une libération rapide de zinc et/ou d'au moins un sel de saccharide sous forme de zinc, très tôt après l'administration du produit. Cette libération permet d'obtenir un effet anti-inflammatoire, anti-infectieux et cicatrisant rapidement après l'administration du produit et d'augmenter la rémanence *in vivo* du produit.

On entend par « produit injectable » au sens de la présente invention, un produit adapté à l'administration par injection. Ainsi, le produit selon l'invention peut être administré selon différents modes d'injection adaptés notamment au site d'injection et à l'effet recherché.

On entend par « produit biocompatible » au sens de la présente invention, tout produit qui est bien toléré par un organisme vivant et qui ne provoque pas de réaction de rejet, de réaction toxique, de lésion ou d'effet nocif sur les fonctions biologiques de ce dernier.

On entend par « produit à libération de zinc et/ou de sels de saccharides sous forme de zinc », tout produit à partir duquel du zinc ou un sel de saccharide sous forme de zinc est diffusé dans l'organisme après administration.

La libération de zinc ou des chaînes de sels de saccharides sous forme de zinc à partir de la matrice selon l'invention peut s'effectuer par simple diffusion (plus ou moins rapide en fonction de la nature et de la densité du réseau de polymères), ou par libération progressive résultant de la dégradation radicalaire et/ou enzymatique de la matrice.

On entend par « zinc », le métal de symbole Zn et de numéro atomique 30. Le zinc est un oligo-élément indispensable à l'homme, puisqu'il joue un rôle majeur dans de nombreux processus physiologiques. Ainsi, bien que les mécanismes d'action soient encore mal élucidés, la supplémentation en zinc diminue significativement les risques d'infection, tel que décrit dans la Demande Internationale WO 98/10773 ou dans l'article de Brooks et al. (The Lancet 366(9490), 2005).

On entend par « saccharide », au sens de la présente invention, aussi bien les monosaccharides, les disaccharides, que les polysaccharides. On entend désigner ici par « polysaccharides », des polymères formés d'un nombre n de monosaccharides identiques ou différents, par liaison glycosidique, avec n supérieur ou égal à 3.

La présence d'au moins un sel de saccharide sous forme de zinc dans les produits selon l'invention présente l'avantage d'optimiser les propriétés cicatrisantes, anti-inflammatoires et anti-infectieuses grâce à une synergie entre le zinc et le saccharide.

Dans le produit injectable selon l'invention, ledit au moins un sel de saccharide sous forme de zinc peut être choisi dans le groupe comprenant le hyaluronate de zinc, le gluconate de zinc, le sel de zinc de la carboxymethylcellulose, le sel de zinc d'un glycosaminoglycane et un mélange de ceux-ci, de préférence le hyaluronate de zinc et le gluconate de zinc et tout préférentiellement le hyaluronate de zinc.

La présence de hyaluronate de zinc dans la matrice présente l'avantage d'optimiser les propriétés cicatrisantes, anti-inflammatoires, anti-infectieuses et hydratantes du produit selon l'invention.

Les propriétés hydratantes de produits selon l'invention sont particulièrement intéressantes, par exemple pour la réjuvénation cutanée.

On entend par « dispersé dans la matrice » tout ion zinc ou tout sel de saccharide sous forme de zinc non lié de manière covalente à la matrice.

En particulier, ledit au moins un sel de saccharide sous forme de zinc est en outre greffé dans la matrice.

On entend par « greffé dans la matrice », au sens de la présente invention, la liaison à la matrice par une liaison covalente.

La présence d'au moins un sel de saccharide sous forme de zinc greffé dans la matrice présente l'avantage de libérer ledit au moins un sel de saccharide sous forme de zinc de façon retardée par rapport à un autre produit selon l'invention dans lequel ledit au moins un sel de saccharide sous forme de zinc est seulement sous forme dispersée dans la matrice. Ainsi, au moins une partie d'au moins un sel de saccharide sous forme de zinc, qui est greffée dans la matrice, est libérée avec une cinétique plus lente que le zinc et/ou que ledit au moins un sel de saccharide sous forme de zinc qui sont dispersés dans la matrice. Cette libération de façon retardée permet d'obtenir un effet anti-inflammatoire, anti-infectieux et cicatrisant prolongé, et d'augmenter la rémanence *in vivo* du produit.

Le taux de greffage, défini comme étant le rapport entre le nombre de moles de motifs d'au moins un sel de saccharide sous forme de zinc greffé et le nombre de moles de motifs de polymère(s) de la matrice, peut être compris entre 1 et 50 %, de préférence entre 5 et 30 % et tout préférentiellement entre 10 et 25 %.

La teneur en zinc sous forme, d'au moins un sel de saccharide sous forme de zinc greffé dans la matrice, peut être compris entre 0,01 et 20 %, de préférence entre 0,05 et 10 % et tout préférentiellement entre 0,1 et 6 % en poids par rapport au poids total de la matrice.

En particulier, ledit au moins un sel de saccharides sous forme de zinc est en outre réticulé dans la matrice.

On entend par « réticulé dans la matrice », au sens de la présente invention, la liaison à la matrice par au moins deux liaisons covalentes.

La présence d'au moins un sel de saccharide sous forme de zinc réticulé dans la matrice présente l'avantage de libérer ledit au moins un sel de saccharide sous forme de zinc de façon retardée par rapport à un autre produit selon l'invention dans lequel ledit au moins un sel de saccharide sous forme de zinc est sous forme dispersée ou greffée dans la matrice. Cette libération de façon retardée permet de prolonger les effets anti-inflammatoires, anti-infectieux et cicatrisants et d'augmenter la rémanence *in vivo* du produit.

La teneur en zinc sous forme, d'au moins un sel de saccharide sous forme de zinc réticulé dans la matrice, est compris entre 0,01 et 30 %, de préférence entre 0,05 et 15 % et tout préférentiellement entre 0,1 et 10 % en poids par rapport au poids total de la matrice.

On entend par « matrice » au sens de la présente invention un réseau de polymères, éventuellement tridimensionnel lorsque les polymères sont réticulés.

On entend par « polymère biocompatible » au sens de la présente invention toute substance composée d'un grand nombre de petites structures moléculaires de faible masse, identiques ou différentes, qui se lient entre elles, pour créer des molécules comportant une masse moléculaire élevée, ladite substance étant bien tolérée par un organisme vivant et ne provoquant pas de réaction de rejet, de réaction toxique, de lésion ou d'effet nocif sur les fonctions biologiques de ce dernier.

Le polymère biocompatible selon l'invention peut être un polymère d'origine naturelle mais également un polymère synthétique.

À titre d'exemples de polymères synthétiques utilisables dans le produit selon l'invention, on peut citer les polymères d'alcool polyvinylique (PVA), le poly(lactide) (PLA) et le poly(lactide-co-glycolide) (PLGA).

De manière préférée, le polymère est un polymère d'origine naturelle choisi dans le groupe comprenant les protéines, les sels de polysaccharides, les polysaccharides et un mélange de ceux-ci, de préférence les sels de polysaccharides et les polysaccharides choisi dans le groupe comprenant le hyaluronate, le sulfate de chondroïtine, les dérivés cellulosiques (tels que la carboxymethylcellulose), le chitosane, l'alginate, la carragénine, le xanthane et un mélange de ceux-ci.

Les polymères d'origine naturelle présentent de nombreux avantages tels qu'une excellente biocompatibilité, des propriétés rhéologiques et hydratantes particulièrement intéressantes pour certains d'entre eux (comme le hyaluronate, certains collagènes), une rémanence *in vivo* variable selon que le polymère est naturellement présent dans notre organisme ou non et des propriétés d'induction de l'adhésion cellulaire pour certains d'entre eux (comme le chitosane). Le mélange de certains de ces polymères dans la matrice selon l'invention permet de coupler leurs propriétés. Ainsi, en faisant varier la composition en polymères de la matrice selon l'invention, il est possible de répondre à divers besoins suivant l'indication souhaitée, notamment médicale ou cosmétique.

La matrice selon l'invention se présente sous une forme choisie dans le groupe comprenant une solution et un gel réticulé. Ces différentes formes peuvent être obtenues par des techniques bien connues de l'Homme du Métier tel que décrit dans la Demande Internationale WO 2006069578 et dans les Brevets US US4963666, US4716154, US5827937 et EP0466300.

Un gel réticulé correspond à un réseau tridimensionnel de polymères réticulés. Lorsque la matrice selon l'invention se présente sous la forme d'un gel réticulé, le taux de réticulation de la matrice, défini comme étant le rapport entre le nombre de moles d'agent réticulant assurant le pontage et le nombre de moles de l'ensemble des chaînes de polymères de la matrice, peut être compris entre 0,1 et 25 %, de préférence entre 1 et 20 % et tout préférentiellement entre 5 et 20 %.

Un taux de réticulation de la matrice compris entre 5 et 20 % présente l'avantage d'obtenir un produit ayant une longue rémanence après administration.

La forme de la matrice est choisie en fonction de l'application pour laquelle est destinée le produit selon l'invention. Ainsi, une matrice sous la forme d'une solution est particulièrement adaptée à des produits injectés superficiellement au niveau de la peau, ou dans un but de lubrification et/ou de supplémentation d'un fluide biologique.

Ainsi, par exemple, afin d'effectuer un traitement de réjuvénation cutanée, les matrices selon l'invention sont préférentiellement des solutions, alors que pour traiter et/ou combler des rides, les matrices utilisées se présentent sous la forme de gels réticulés. En particulier, plus la ride à traiter est profonde, plus les matrices selon l'invention ont avantageusement des taux de réticulation élevés.

Dans le cas des produits utilisés pour la chirurgie de la cataracte qui doivent à la fois recouvrir les tissus et être faciles à retirer en fin de chirurgie, les matrices selon l'invention se présentent avantageusement sous forme de solutions, notamment cohésives, ou de gels ayant un faible taux de réticulation.

Dans le domaine dentaire, dans le cas du traitement de maladies parodontales pour un bon maintien entre la gencive et la dent, les matrices selon l'invention se présentent sous la forme de gels réticulés.

Afin d'augmenter l'effet d'« absorption des chocs » et de lutter contre la douleur tout en conservant l'effet de lubrification, la supplémentation du liquide synovial peut se faire à l'aide d'un produit selon l'invention dont la matrice se présente sous la forme d'un gel réticulé ayant un faible taux de réticulation.

Les produits selon l'invention présentent l'avantage de pouvoir obtenir un effet anti-inflammatoire, anti-infectieux et cicatrisant plus ou moins prolongé et une rémanence *in vivo* du produit plus ou moins longue, en fonction de l'application visée, en modulant la cinétique de libération du zinc et/ou d'au moins un sel de saccharide sous forme de zinc. Cette libération est notamment dépendante de la teneur en zinc et ou d'au moins un sel de saccharide sous forme de zinc dispersé et greffé et/ou réticulé dans la matrice.

Les produits selon l'invention présentent ainsi l'avantage que la rémanence *in vivo* du produit n'est pas uniquement dépendante de la forme de la matrice et en particulier du taux de réticulation de la matrice.

Par ailleurs, la rémanence *in vivo* des produits selon l'invention peut être modulée par la forme de la matrice, en particulier par la variation du taux de réticulation la matrice. Ainsi, plus le taux de réticulation de la matrice est élevé, plus la rémanence du produit selon l'invention est longue.

Les produits selon l'invention peuvent présenter divers degrés de viscosité et d'élasticité, en fonction de divers paramètres tels que la concentration en polymères, le taux de réticulation, la nature de l'excipient, le procédé d'obtention. La viscosité à taux de cisaillement nul à température ambiante des produits sous forme de solution selon la présente invention peut atteindre jusqu'à 1500 Pa.s. Le module élastique mesuré à l'aide d'un rhéomètre, en mode dynamique, des produits selon l'invention se présentant sous forme de gels viscoélastiques (gels réticulés) peut atteindre jusqu'à 200 Pa.

Le degré de viscosité et d'élasticité est choisi en fonction de l'application pour laquelle est destiné le produit selon l'invention.

Un produit selon l'invention ayant des propriétés viscoélastiques est ainsi utile par exemple pour soulager des douleurs liées au fonctionnement d'articulation et lors de chirurgies ophtalmiques telles que la cataracte pour protéger les différents tissus de l'oeil.

Selon un second aspect, l'invention a pour objet une composition cosmétique ou pharmaceutique, comprenant un produit selon l'invention.

On entend par « composition cosmétique » au sens de la présente invention toute composition destinée à des fins esthétiques. Les compositions cosmétiques au sens de la présente invention incluent les compositions topiques mais aussi injectables, de préférence injectables.

Les compositions selon l'invention peuvent comprendre en outre au moins un agent choisi dans le groupe comprenant un agent antioxydant, un autre agent cicatrisant, un autre agent anti-inflammatoire et un mélange de ceux-ci, comme produit de combinaison pour une utilisation simultanée, séparée et/ou étalée dans le temps.

Lesdits agents antioxydants, cicatrisants et anti-inflammatoires utilisables dans les compositions selon l'invention peuvent être aisément déterminés par l'Homme du Métier. Ledit agent antioxydant peut notamment être choisi parmi les vitamines ayant un effet antioxydant telles que la vitamine A, la vitamine C, la vitamine E et les dérivés desdites vitamines. Ledit autre agent cicatrisant peut notamment être de l'alginate. Ledit autre agent anti-inflammatoire peut être choisi parmi les agents anti-inflammatoires non stéroïdiens, et les corticoïdes.

Selon un troisième aspect, l'invention a pour objet un dispositif médical comprenant un produit selon l'invention.

On entend par « dispositif médical », au sens de la présente invention, tout produit destiné à être utilisé chez l'homme à des fins médicales.

Ainsi, un dispositif médical selon l'invention peut, par exemple, être constitué d'une seringue pré-remplie, comprenant la dose d'injection unitaire d'un produit selon l'invention. Un dispositif médical selon l'invention peut également être un flacon pré-rempli comprenant la dose unitaire d'un produit selon l'invention, permettant l'administration dudit produit sous la forme de goutte, telle que des gouttes ophtalmiques. En particulier, cette dose unitaire peut varier de 0,1 à 5 mL, de préférence de 0,1 à 2 mL.

De manière préférée, les dispositifs médicaux selon l'invention sont à usage unique.

Les compositions, produits et dispositifs médicaux selon l'invention sont de préférence administrés par injection ou sous la forme de gouttes telles que des gouttes ophtalmiques.

Le mode d'injection sera déterminé notamment en fonction du site d'injection et de l'effet recherché. Les injections peuvent être notamment sous-cutanées, intradermique, intra-articulaire, oculaires, intra-musculaire, intra-periostale ou encore réalisées au niveau buccal. Les injections peuvent être effectuées selon une technique "manuelle" avec seringue et aiguille ou selon une technique "assistée" avec l'aide d'un injecteur électronique.

Le taux d'injection et le taux d'administration sous forme de gouttes du produit, des compositions et des dispositifs médicaux selon l'invention peut varier en fonction notamment du site d'administration, et/ou de l'effet recherché. Ainsi, le taux d'injection peut varier entre 1 et 10 injections par jour. Le taux d'administration sous forme de gouttes du produit, des compositions et des dispositifs médicaux selon l'invention peut varier par exemple entre 1 et 15 gouttes par jour. De préférence chaque injection ou goutte comprend de 0,1 à 5 mL, de préférence de 0,1 à 2 mL de produit selon l'invention.

Par exemple, dans le cas de réjuvénation cutanée, notamment du visage, chaque injection peut comprendre 1 mL de produit selon l'invention. Dans le cas de dégénérescence articulaire, le produit selon l'invention peut être injecté 3 fois à raison de 1 semaine d'intervalle avec 2 mL de produit par injection. Lors de chirurgies ophtalmiques telles que la cataracte, le produit selon l'invention peut être administré à des doses de 0,5 à 2 mL pour protéger les différents tissus de l'oeil.

Les compositions, les produits et les dispositifs médicaux selon l'invention peuvent être utilisés en mésothérapie. La mésothérapie est une technique basée sur l'administration multiples de faibles doses de produits sous la peau. Cette technique est aussi bien utilisée à des fins esthétiques (cellulite, rides) qu'à des fins médicales (arthrose, mal de dos, troubles douloureux après traitement chirurgical).

Selon un quatrième aspect, l'invention a pour objet l'utilisation cosmétique d'un produit selon l'invention, en tant qu'agent cosmétique pour combler ou augmenter le volume d'un tissu biologique ou remplacer ou supplémenter un fluide biologique, dans une composition cosmétique.

En particulier, la composition cosmétique selon l'invention est destinée à :
(i) prévenir et/ou lutter contre les signes du vieillissement cutané ; et/ou
(ii) améliorer la texture de la peau ; et/ou
(iii) raviver l'éclat de la peau ; et/ou
(iv) effacer et traiter les rides et/ou ridules de la peau ; et/ou
(v) augmenter les volumes du visage ou du corps ; et/ou
(vi) redéfinir les contours du visage ou du corps.

Par exemple, les compositions cosmétiques selon l'invention peuvent être destinés à augmenter le volume de la lèvre supérieure chez des malades atteints de prognathie mandibulaire.

Selon un cinquième aspect, l'invention a pour objet un procédé de traitement cosmétique destiné à
(i) prévenir et/ou lutter contre les signes du vieillissement cutané ; et/ou
(ii) améliorer la texture de la peau ; et/ou
(iii) raviver l'éclat de la peau ; et/ou
(iv) effacer et traiter les rides et/ou ridules de la peau ; et/ou
(v) augmenter les volumes du visage ou du corps ; et/ou
(vi) redéfinir les contours du visage ou du corps ;
comprenant l'injection dans les zones concernées d'un produit selon l'invention ou d'une composition cosmétique selon l'invention.

Selon un autre aspect, l'invention a pour objet, l'utilisation d'un produit selon l'invention pour la préparation d'un médicament ou d'un dispositif médical, ayant une activité anti-inflammatoire locale et cicatrisante, destiné à :
(i) combler ou remplacer un tissu biologique ; et/ou
(ii) augmenter le volume dudit tissu biologique ; et/ou
(iii) supplémenter ou remplacer un fluide biologique.

On entend par « médicament ou dispositif médical, ayant une activité anti-inflammatoire locale », au sens de la présente invention, tout médicament ou dispositif médical ayant une action anti-inflammatoire ciblée dans la zone d'administration du médicament ou du dispositif médical selon l'invention.

Selon un autre aspect, l'invention a pour objet, l'utilisation d'un produit selon l'invention, pour la préparation d'un médicament ou d'un dispositif médical, ayant une activité anti-inflammatoire locale et cicatrisante, destiné à la prévention et/ou au traitement de pathologies pouvant être améliorées ou évitées par :
(i) le comblement ou le remplacement d'un tissu biologique ; et/ou
(ii) l'augmentation de volume dudit tissu biologique ; et/ou
(iii) la supplémentation ou le remplacement d'un fluide biologique.

De telles pathologies peuvent être choisies dans le groupe comprenant les dégénérescences articulaires telles que l'arthrose, l'arthrite, les paradontoses, les pathologies neurologiques, les pathologies orthopédiques.

Selon un autre aspect, l'invention a pour objet l'utilisation d'un produit selon l'invention, pour la préparation d'un médicament ou d'un dispositif médical, ayant une activité anti-inflammatoire locale et cicatrisante, destiné à la prévention et/ou au traitement de pathologies ou de troubles liés au vieillissement ou consécutifs à un acte chirurgical.

À titre d'exemples de troubles liés au vieillissement, on peut citer les troubles articulaires tels que l'arthrose, l'arthrite.

À titre d'exemples de troubles consécutifs à un acte chirurgical, on peut citer l'opacification de la cornée, les lésions tissulaires, les troubles liés à la pression oculaire dans le cas de chirurgie ophtalmique.

Les actes chirurgicaux comprennent les actes ophtalmiques. Ainsi les médicaments ou dispositifs médicaux selon l'invention peuvent être utilisés en tant que viscoélastique pour diverses chirurgies ophtalmiques, telle que la cataracte pour protéger les différents tissus de l'oeil. En outre, la présence de zinc dans les produits de la présente invention utilisés en ophtalmologie est particulièrement intéressante du fait de ses propriétés anti-oxydantes et de son rôle majeur dans divers processus physiologiques oculaires.

À titre d'exemple, les médicaments et les dispositifs médicaux selon l'invention peuvent être utilisés pour supplémenter ou remplacer le liquide synovial dans le cas de pertes de liquide synovial par diffusion et/ou endommagement notamment de l'articulation du genou. Ce liquide clair, translucide et visqueux sécrété par les membranes synoviales permet de lubrifier les articulations, les bourses séreuses et les tendons, protégeant ainsi le cartilage des stress mécaniques. Les médicaments et les dispositifs médicaux selon l'invention peuvent ainsi être utilisés pour améliorer la lubrification et l'absorption des chocs. En particulier, en raison de leur viscoélasticité, les produits selon l'invention dont la matrice se présente sous la forme d'un gel, peuvent en outre apporter une sensation de soulagement lors du fonctionnement de l'articulation.

Ainsi, les médicaments et les dispositifs médicaux selon l'invention peuvent être destinés à supplémenter le liquide synovial par injection intra-articulaire, notamment dans le cadre de la prévention et/ou du traitement d'une arthrose telle que la gonarthrose, la rhizarthrose ou de l'arthrite.

Les médicaments et les dispositifs médicaux selon l'invention peuvent être destinés à remplacer ou supplémenter l'humeur aqueuse (lors de la chirurgie de la cataracte ou dans le cadre de chirurgie réfractive) ou l'humeur vitrée (dans le cas d'une vitrectomie).

Les médicaments et les dispositifs médicaux selon l'invention peuvent également être avantageux par rapport aux chirurgies classiques dans le remplacement de tissu biologique, par exemple dans des maladies dégénératives du disque intervertébral pour remplacer le nucleus pulposus.

Selon un autre aspect, l'invention a pour objet un agent de comblement ou de remplacement ou d'augmentation de volume d'un tissu biologique, ayant une activité anti-inflammatoire locale et cicatrisante, comprenant un produit selon l'invention.

Selon un autre aspect, l'invention a pour objet un agent de remplacement ou supplémentant un fluide biologique, ayant une activité anti-inflammatoire locale et cicatrisante, comprenant un produit selon l'invention.

D'autres avantages et caractéristiques de l'invention apparaîtront au regard des exemples qui suivent.

Ces exemples sont donnés à titre illustratif et non limitatif.

### Exemples

### I. Exemple 1 : Exemples de procédés de préparation de produits selon l'invention, leurs effets et leurs applications

Les exemples 1, 5 et 6 concernent la préparation d'un produit dans lequel le sel de saccharide sous forme de zinc est dispersé dans la matrice.

Les autres exemples concernent la préparation d'un produit dans lequel le sel de saccharide sous forme de zinc est réticulé et/ou greffé dans la matrice.

### I.1 1^{er} exemple de procédé de préparation d'un produit

1 gramme (g) de hyaluronate de Zinc (Zn) est ajouté à 200 mL d'une solution tamponnée de hyaluronate de sodium (2MDa) à 10 mg/g.

La solution obtenue est homogénéisée et allicotée par mise en seringue stérile.

Le produit obtenu disponible dans des seringues stériles pré-remplies, est particulièrement adapté:
- pour la réjuvénation du tissu cutané par la technique de mésothérapie (nombreuses injections de faibles quantités de produit sur la zone cutanée à traiter comme le visage, le décolleté, les mains, le cou ...);
- pour le traitement d'une arthrose (gonarthrose, rhizarthrose ...), par viscosupplémentation (injection intra-articulaire);
- en tant que viscoélastique pour divers chirurgies ophtalmiques, notamment la chirurgie de la cataracte, pour protéger les différents tissus de l'oeil.

Ce produit, biocompatible et résorbable permet notamment:
- de limiter les désagréments subis par le patient à cause de l'inflammation par l'injection du produit,
- d'améliorer la cicatrisation au niveau du site d'injection,
- d'accroître la rémanence du produit.

### I.2 2^{nd} exemple de procédé de préparation d'un produit selon l'invention

2g de hyaluronate de sodium (NaHA) 2-2,5MDa sont progressivement ajoutés à 20ml d'une solution alcaline (pH=12,5). L'hydratation progressive du NaHA est réalisée par une homogénéisation pendant 1h30 à T=20°C. 100µl de BDDE sont ensuite ajoutés à la solution et l'ensemble est placé 2h à 50°C.

Le gel obtenu est neutralisé par addition de HCl et dilué par addition de tampon phosphate de pH=7, pour obtenir une concentration en hyaluronate de 30mg/ml. Le produit est alors dialysé , à l'aide d'une solution tampon (pH=7 et osmolalité de 280 milliOsmole/L) pour éliminer toutes les impuretés.

500mg de hyaluronate de Zn sont alors ajoutés et intégrés par homogénéisation à la matrice réticulée.

Le gel est allicoté, mis en seringue, stérilisé.

Le produit obtenu disponible dans des seringues stériles pré-remplies, est particulièrement adapté pour l'injection intradermique ou sous-cutanée, pour le comblement de rides fines (peu profondes) ou moyennes ou le remodelage du visage.

Ce produit, biocompatible et résorbable permet notamment:
- de limiter les désagréments subis par le patient à cause de l'inflammation par l'injection du produit;
- d'améliorer la cicatrisation au niveau du site d'injection ;

Ce produit permet de libérer le hyaluronate de zinc et/ou le zinc de façon retardée par rapport au produit de l'exemple 1, ce qui permet d'obtenir un effet anti-inflammatoire, anti-infectieux et cicatrisant prolongé par rapport aux effets obtenus avec le produit selon l'exemple 1. Ceci permet également d'accroître la rémanence du produit, par rapport au produit décrit dans l'exemple 1.

### I.3 3^{ème} exemple de procédé de préparation d'un produit selon l'invention

2g de hyaluronate de sodium (NaHA) 2-2,5MDa sont progressivement ajoutés à 20ml d'une solution alcaline (pH=12,5). L'hydratation progressive du NaHA est réalisée par une homogénéisation pendant 1h30 à T=20°C. 220µl de BDDE sont ensuite ajoutés à la solution et l'ensemble est placé 2h à 50°C.

Le gel obtenu est neutralisé par addition de HCl et dilué par addition de tampon phosphate pour obtenir une concentration en hyaluronate de 30mg/ml. Le produit est alors dialysé pour éliminer toutes les impuretés.

500mg de hyaluronate de Zn sont alors ajoutés et intégrés par homogénéisation à la matrice réticulée.

Le gel est allicoté, mis en seringue, stérilisé.

Le produit obtenu disponible dans des seringues stériles pré-remplies, est particulièrement adapté pour l'injection intradermique, intra-musculaire, intra-périostale ou sous-cutanée, pour le comblement de rides, notamment profondes, ou le remodelage du visage ou du corps.

Ce produit, biocompatible et résorbable permet notamment:
- de limiter les désagréments subis par le patient à cause de l'inflammation par l'injection du produit,
- d'améliorer la cicatrisation au niveau du site d'injection,
- d'accroître la rémanence du produit.

Ce produit permet de libérer le hyaluronate de zinc et/ou le zinc de façon retardée par rapport au produit de l'exemple 2 (en raison du taux de réticulation plus élevé de la matrice), ce qui permet d'obtenir un effet anti-inflammatoire, anti-infectieux et cicatrisant prolongé par rapport aux effets obtenus avec le produit selon l'exemple 2. Ceci permet également d'accroître la rémanence du produit, par rapport au produit décrit dans l'exemple 2 et d'être adapté à une injection intradermique ou sous-cutanée plus profonde que le produit décrit dans l'exemple 2.

### I.4 4^{ème} exemple de procédé de préparation d'un produit selon l'invention

2 grammes de carboxymethylcellulose sont progressivement ajoutés à 20 ml d'une solution alcaline (NaOH ou Zn(OH)₂) (pH=12,5). L'hydratation progressive de la carboxymethylcellulose est réalisée par une homogénéisation pendant 1h30 à T=20°C. 220µl de BDDE (1,4-Butanediol diglycidylether) sont ensuite ajoutés à la solution et l'ensemble est placé 2h à 50°C.

Le gel obtenu est neutralisé par addition de HCl et dilué par addition de tampon phosphate de pH=7. Le produit est alors dialysé, à l'aide d'une solution tampon (pH=7 et osmolalité de 180 milliOsmole/L) pour éliminer toutes les impuretés.

500 mg de hyaluronate de Zn sont alors ajoutés et intégrés par homogénéisation à la matrice de carboxymethylcellulose réticulée.

Le gel est allicoté, mis en seringue, stérilisé.

Le produit obtenu disponible dans des seringues stériles pré-remplies, est particulièrement adapté pour l'injection intradermique, intra-musculaire, intra-périostale ou sous-cutanée, pour le comblement de rides ou le remodelage du visage ou du corps.

Ce produit, biocompatible permet notamment:
- de limiter les désagréments subis par le patient à cause de l'inflammation par l'injection du produit,
- d'améliorer la cicatrisation au niveau du site d'injection,
- d'accroître la rémanence du produit par rapport au produit décrit dans l'exemple 3.

### I.5 5^{ème} exemple de procédé de préparation d'un produit

Une solution est préparée à base des sels ZnCl2 et NaCl dilués dans de l'eau pure, afin d'obtenir un excipient caractérisé par un pH de 6,9, une osmolarité de 300 milliOsmole/L, et une concentration en zinc de 1,18mg/ml.

1,4g de hyaluronate de sodium 2MDa est ajouté à 100ml de cet excipient et le tout est homogénéisé. Cette solution est ensuite mise en seringue après une filtration stérilisante.

Le produit obtenu, prêt à être injecté, est particulièrement adapté pour:
- la réjuvénation du tissu cutané, par la technique de mésothérapie
- le traitement d'une arthrose par viscosupplémentation
- en tant que viscoélastique pour un usage temporaire lors de diverses chirurgies ophtalmiques.

Ce produit compatible et résorbable permet notamment de:
- limiter les désagréments, résultats de l'inflammation engendrés par l'acte d'injection, notamment lors de la réjuvénation cutanée
- assurer une bonne hydratation du tissu cutané, une bonne protection des tissues oculaires lors d'une chirurgie
- assurer une viscosupplémentation efficace après injection dans la poche synoviale, et de limiter l'inflammation locale

### I.6 6^{ème} exemple de procédé de préparation d'un produit

1g de hyaluronate de sodium 2MDa est ajouté à 100ml d'un tampon phosphate (pH=6,8 et Osmolarité: 240 milliOsmole/L) et le tout est homogénéisé. Puis du ZnCl₂ est ajouté à cette solution visqueuse (concentration en zinc: 1mg/ml). Cette solution est ensuite mise en seringue après une filtration stérilisante.

Le produit obtenu, prêt à être injecté, est particulièrement adapté pour:
- la réjuvénation du tissu cutané, par la technique de mésothérapie
- le traitement d'une arthrose par viscosupplémentation
- en tant que viscoélastique pour un usage temporaire lors de diverses chirurgies ophtalmiques.

## Revendications

1. Produit injectable biocompatible à libération de zinc et/ou d'au moins un sel de saccharide sous forme de zinc, comprenant :
- une matrice comprenant au moins un polymère biocompatible choisi dans le groupe consistant en les sels de polysaccharides, les polysaccharides et un mélange de ceux-ci ; et
- au moins un sel de saccharide sous forme de zinc;
dans lequel ledit au moins un sel de saccharide sous forme de zinc est dispersé et réticulé dans la matrice ; et
dans lequel la teneur en zinc sous forme d'au moins un sel de saccharide sous forme de zinc dispersé dans la matrice, est comprise entre 0,01 et 10 %, de préférence entre 0,05 et 5 % et tout préférentiellement entre 0,1 et 3 % en poids par rapport au poids total de la matrice ; et
dans lequel la teneur en zinc sous forme, d'au moins un sel de saccharide sous forme de zinc réticulé dans la matrice, est comprise entre 0,01 et 30 %, de préférence entre 0,05 et 15 % et tout préférentiellement entre 0,1 et 10 % en poids par rapport au poids total de la matrice.

2. Produit selon la revendication 1, **caractérisé en ce que** ledit au moins un sel de saccharide sous forme de zinc est en outre greffé dans la matrice.

3. Produit selon la revendication 2, **caractérisé en ce que** le taux de greffage, défini comme étant le rapport entre le nombre de moles de motifs d'au moins un sel de saccharide sous forme de zinc greffé et le nombre de moles de motifs de polymère(s) de la matrice, est compris entre 1 et 50 %, de préférence entre 5 et 30 % et tout préférentiellement entre 10 et 25 %.

4. Produit selon l'une des revendications 2 ou 3, **caractérisé en ce que** la teneur en zinc sous forme, d'au moins un sel de saccharide sous forme de zinc greffé dans la matrice, est comprise entre 0,01 et 20 %, de préférence entre 0,05 et 10 % et tout préférentiellement entre 0,1 et 6 % en poids par rapport au poids total de la matrice.

5. Produit selon l'une quelconque des revendications 1 à 4, **caractérisé en ce que** ledit au moins un sel de saccharide sous forme de zinc est choisi dans le groupe consistant en le hyaluronate de zinc, le gluconate de zinc, le sel de zinc de la carboxymethylcellulose, le sel de zinc d'un glycosaminoglycane et un mélange de ceux-ci.

6. Produit selon l'une quelconque des revendications 1 à 5, **caractérisé en ce que** les sels de polysaccharides et les polysaccharides sont choisis dans le groupe consistant en le hyaluronate, le sulfate de chondroïtine, les dérivés cellulosiques, le chitosane, l'alginate, la carragénine, le xanthane et un mélange de ceux-ci.

7. Produit selon l'une quelconque des revendications 1 à 6, **caractérisé en ce que** la matrice se présente sous une forme choisie dans le groupe comprenant une solution et un gel réticulé.

8. Produit selon la revendication 7, **caractérisé en ce que** la matrice se présente sous la forme d'un gel réticulé dans lequel le taux de réticulation de la matrice, défini comme étant le rapport entre le nombre de moles d'agent réticulant assurant le pontage et le nombre de moles de l'ensemble des chaînes de polymères de la matrice, est compris entre 0,1 et 25 %, de préférence entre 1 et 20 % et tout préférentiellement entre 5 et 20 %.

9. Composition cosmétique ou pharmaceutique, comprenant un produit selon l'une quelconque des revendications 1 à 8.

10. Composition selon la revendication 9, **caractérisée en ce qu'**elle comprend en outre au moins un agent choisi dans le groupe consistant en un agent antioxydant, un autre agent cicatrisant, un autre agent anti-inflammatoire et un mélange de ceux-ci, comme produit de combinaison pour une utilisation simultanée, séparée et/ou étalée dans le temps.

11. Utilisation cosmétique d'un produit selon l'une quelconque des revendications 1 à 8
dans lequel la teneur en zinc sous forme d'au moins un sel de saccharide sous forme de zinc dispersé dans la matrice, est comprise entre 0,01 et 10 %, de préférence entre 0,05 et 5 % et tout préférentiellement entre 0,1 et 3 % en poids par rapport au poids total de la matrice ;
en tant qu'agent cosmétique pour combler ou augmenter le volume d'un tissu biologique ou remplacer ou supplémenter un fluide biologique, dans une composition cosmétique, ladite composition cosmétique étant destinée à :
(i) prévenir et/ou lutter contre les signes du vieillissement cutané ; et/ou
(ii) améliorer la texture de la peau ; et/ou
(iii) raviver l'éclat de la peau ; et/ou
(iv) effacer et traiter les rides et/ou ridules de la peau ; et/ou
(v) augmenter les volumes du visage ou du corps ; et/ou
(vi) redéfinir les contours du visage ou du corps.

12. Procédé de traitement cosmétique destiné à
(i) prévenir et/ou lutter contre les signes du vieillissement cutané ; et/ou
(ii) améliorer la texture de la peau ; et/ou
(iii) raviver l'éclat de la peau ; et/ou
(iv) effacer et traiter les rides et/ou ridules de la peau ; et/ou
(v) augmenter les volumes du visage ou du corps ; et/ou
(vi) redéfinir les contours du visage ou du corps ;
comprenant l'injection dans les zones concernées d'un produit tel que défini selon la revendication 11 ou d'une composition cosmétique selon la revendication 9.

13. Produit selon l'une quelconque des revendications 1 à 8, pour son utilisation en tant que médicament ayant une activité antiinflammatoire locale et cicatrisante, destiné à :
(i) combler ou remplacer un tissu biologique ; et/ou
(ii) augmenter le volume dudit tissu biologique ; et/ou
(iii) supplémenter ou remplacer un fluide biologique.

14. Produit selon l'une quelconque des revendications 1 à 8, pour son utilisation en tant que médicament ayant une activité anti-inflammatoire locale et cicatrisante, destiné à la prévention et/ou au traitement de pathologies pouvant être améliorées ou évitées par :
(i) le comblement ou le remplacement d'un tissu biologique ; et/ou
(ii) l'augmentation de volume dudit tissu biologique ; et/ou
(iii) la supplémentation ou le remplacement d'un fluide biologique.

15. Produit pour son utilisation selon la revendication 14, **caractérisée en ce que** les pathologies sont choisies dans le groupe consistant en les dégénérescences articulaires telles que l'arthrose, l'arthrite, les paradontoses, les pathologies neurologiques, les pathologies orthopédiques.

16. Produit selon l'une quelconque des revendications 1 à 8, pour son utilisation en tant que médicament ayant une activité antiinflammatoire locale et cicatrisante, destiné à la prévention et/ou au traitement de pathologies ou de troubles liés au vieillissement ou consécutifs à un acte chirurgical.

17. Agent de comblement ou de remplacement ou d'augmentation de volume d'un tissu biologique, ayant une activité anti-inflammatoire locale et cicatrisante, comprenant un produit selon l'une quelconque des revendications 1 à 8.

18. Agent de remplacement ou supplémentant un fluide biologique, ayant une activité anti-inflammatoire locale et cicatrisante, comprenant un produit selon l'une quelconque des revendications 1 à 8.

## Patentansprüche

1. Biokompatibles injizierbares Produkt mit Freisetzung von Zink und/oder mindestens einem Saccharidsalz in Form von Zink, umfassend:
- eine Matrix, umfassend mindestens ein biokompatibles Polymer, ausgewählt aus der Gruppe, bestehend aus den Polysaccharidsalzen, Polysacchariden und einer Mischung dieser; und
- mindestens ein Saccharidsalz in Form von Zink;
wobei das mindestens eine Saccharidsalz in Form von Zink in der Matrix dispergiert und vernetzt ist; und
wobei der Zinkgehalt in Form von mindestens einem Saccharidsalz in Form von Zink, dispergiert in der Matrix, zwischen 0,01 und 10 %, vorzugsweise zwischen 0,05 und 5 % und am meisten bevorzugt zwischen 0,1 und 3 Gew.-% mit Bezug auf das Gesamtgewicht der Matrix liegt; und
wobei der Zinkgehalt in Form von mindestens einem Saccharidsalz in Form von Zink, vernetzt in der Matrix, zwischen 0,01 und 30 %, vorzugsweise zwischen 0,05 und 15 % und am meisten bevorzugt zwischen 0,1 und 10 Gew.-% mit Bezug auf das Gesamtgewicht der Matrix liegt.

2. Produkt nach Anspruch 1, **dadurch gekennzeichnet, dass** das mindestens eine Saccharidsalz in Form von Zink außerdem in der Matrix gepfropft ist.

3. Produkt nach Anspruch 2, **dadurch gekennzeichnet, dass** die Pfropfrate, definiert als das Verhältnis zwischen der Anzahl der Mole mit Muster von mindestens einem Saccharidsalz in Form von gepfropftem Zink und der Anzahl der Mole mit Polymermuster der Matrix zwischen 1 und 50 %, vorzugsweise zwischen 5 und 30 % und am meisten bevorzugt zwischen 10 und 25 % liegt.

4. Produkt nach Anspruch 2 oder 3, **dadurch gekennzeichnet, dass** der Zinkgehalt in Form von mindestens einem Saccharidsalz in Form von Zink, gepfropft in der Matrix, zwischen 0,01 und 20 %, vorzugsweise zwischen 0,05 und 10 % und am meisten bevorzugt zwischen 0,1 und 6 Gew.-% mit Bezug auf das Gesamtgewicht der Matrix liegt.

5. Produkt nach einem der Ansprüche 1 bis 4, **dadurch gekennzeichnet, dass** das mindestens eine Saccharidsalz in Form von Zink ausgewählt ist aus der Gruppe, bestehend aus Zinkhyaluronat, Zinkgluconat, Carboxymethylcellulose-Zinksalz, Zinksalz eines Glycosaminoglycans und eine Mischung dieser.

6. Produkt nach einem der Ansprüche 1 bis 5, **dadurch gekennzeichnet, dass** die Polysaccharidsalze und die Polysaccharide ausgewählt sind aus der Gruppe, bestehend aus Hyaluronat, Chondroitinsulfat, Zellulosederivaten, Chitosan, Alginat, Carragenin, Xanthan und eine Mischung dieser.

7. Produkt nach einem der Ansprüche 1 bis 6, **dadurch gekennzeichnet, dass** die Matrix eine Form aufweist, ausgewählt aus der Gruppe, umfassend eine Lösung und ein vernetztes Gel.

8. Produkt nach Anspruch 7, **dadurch gekennzeichnet, dass** die Matrix die Form eines vernetzten Gels aufweist, wobei der Vernetzungsgrad der Matrix, definiert als die Beziehung zwischen der Anzahl von Molen des Vernetzungsmittels, die die Brückenbildung sicherstellen, und der Anzahl der Mole der Gesamtheit der Polymerketten der Matrix zwischen 0,1 und 25 %, vorzugsweise zwischen 1 und 20 % und am meisten bevorzugt zwischen 5 und 20 % liegt.

9. Kosmetische oder pharmazeutische Zusammensetzung, umfassend ein Produkt nach einem der Ansprüche 1 bis 8.

10. Zusammensetzung nach Anspruch 9, **dadurch gekennzeichnet, dass** sie außerdem mindestens ein Mittel umfasst, ausgewählt aus der Gruppe, bestehend aus einem Antioxidationsmittel, einem weiteren Wundheilmittel, einem weiteren entzündungshemmenden Mittel und einer Mischung dieser als Kombinationsprodukt für eine gleichzeitige, getrennte und/oder zeitlich versetzte Verwendung.

11. Kosmetische Verwendung eines Produkts nach einem der Ansprüche 1 bis 8
wobei der Zinkgehalt in Form von mindestens einem Saccharidsalz in Form von Zink, dispergiert in der Matrix, zwischen 0,01 und 10 %, vorzugsweise zwischen 0,05 und 5 % und am meisten bevorzugt zwischen 0,1 und 3 Gew.-% mit Bezug auf das Gesamtgewicht der Matrix liegt;
als kosmetisches Mittel, um das Volumen eines biologischen Gewebes zu füllen oder zu erhöhen oder ein biologisches Fluid in einer kosmetischen Zusammensetzung zu ersetzen oder zu ergänzen, wobei die kosmetische Zusammensetzung ausgelegt ist, um:
(i) den Zeichen der Alterung der Haut vorzubeugen und/oder gegen diese zu kämpfen; und/oder
(ii) die Textur der Haut zu verbessern; und/oder
(iii) den Glanz der Haut wiederzubeleben; und/oder
(iv) die Falten und/oder Fältchen der Haut zu beseitigen und zu behandeln; und/oder
(v) die Volumina des Gesichts oder des Körpers zu erhöhen; und/oder
(vi) die Umrisse des Gesichts oder des Körpers neu zu definieren.

12. Verfahren zur kosmetischen Behandlung, ausgelegt, um
(i) den Zeichen der Alterung der Haut vorzubeugen und/oder gegen diese zu kämpfen; und/oder
(ii) die Textur der Haut zu verbessern; und/oder
(iii) den Glanz der Haut wiederzubeleben; und/oder
(iv) die Falten und/oder Fältchen der Haut zu beseitigen und zu behandeln; und/oder
(v) die Volumina des Gesichts oder des Körpers zu erhöhen; und/oder
(vi) die Umrisse des Gesichts oder des Körpers neu zu definieren;
umfassend die Injektion in die betroffenen Bereiche eines Produkts, wie nach Anspruch 11 definiert, oder einer kosmetischen Zusammensetzung nach Anspruch 9.

13. Produkt nach einem der Ansprüche 1 bis 8 für seine Verwendung als Medikament mit einer lokalen und wundheilenden entzündungshemmenden Aktivität, die ausgelegt ist, um:
(i) ein biologisches Gewebe zu füllen oder zu ersetzen; und/oder
(ii) das Volumen des biologischen Gewebes zu erhöhen; und/oder
(iii) ein biologisches Fluid zu ersetzen oder zu ergänzen.

14. Produkt nach einem der Ansprüche 1 bis 8 für seine Verwendung als Medikament mit einer lokalen und wundheilenden entzündungshemmenden Aktivität, ausgelegt zur Verhinderung und/oder Behandlung von Krankheiten, die verbessert oder verhindert werden können durch:
(i) die Füllung oder Ersetzung eines biologischen Gewebes; und/oder
(ii) die Erhöhung des Volumens des biologischen Gewebes; und/oder
(iii) die Ergänzung oder den Ersatz eines biologischen Fluids.

15. Produkt für seine Verwendung nach Anspruch 14, **dadurch gekennzeichnet, dass** die Krankheiten ausgewählt sind aus der Gruppe, bestehend aus den Gelenkdegenerationen wie z.B. der Arthrose, der Arthritis, den Paradontosen, den neurologischen Krankheiten den orthopädischen Krankheiten.

16. Produkt nach einem der Ansprüche 1 bis 8 für seine Verwendung als Medikament mit einer lokalen und wundheilenden entzündungshemmenden Aktivität, ausgelegt zur Prävention und/oder Behandlung von Krankheiten oder Störungen mit Bezug auf die Alterung oder in der Folge eines chirurgischen Eingriffs.

17. Mittel zum Füllen oder Ersetzen oder Erhöhen des Volumens eines biologischen Gewebes mit einer lokalen und wundheilenden entzündungshemmenden Aktivität, umfassend ein Produkt nach einem der Ansprüche 1 bis 8.

18. Mittel zum Ersetzen oder Ergänzen eines biologischen Fluids mit einer lokalen und wundheilenden entzündungshemmenden Aktivität, umfassend ein Produkt nach einem der Ansprüche 1 bis 8.

## Claims

1. Biocompatible injectable product with zinc and/or at least one zinc-form saccharide-salt release, comprising:
- a matrix comprising at least one biocompatible polymer chosen from the group comprising polysaccharide salts, polysaccharides and a mixture thereof; and
- at least one saccharide salt in the form of zinc;
wherein said at least one saccharide salt in the form of zinc is dispersed and cross-linked in the matrix; and
wherein the content of zinc in the form of at least one saccharide salt in the form of zinc dispersed in the matrix, is between 0.01 and 10%, preferably between 0.05 and 5% and very preferably between 0.1 and 3% by weight with respect to the total weight of the matrix; and
wherein the content of zinc in the form of at least one saccharide salt in the form of cross-linked zinc in the matrix, is between 0.01 and 30%, preferably between 0.05 and 15% and very preferably between 0.1 and 10% by weight with respect to the total weight of the matrix.

2. Product according to claim 1, **characterised in that** said at least one saccharide salt in the form of zinc is furthermore grafted in the matrix.

3. Product according to claim 2, **characterised in that** the grafting ratio, defined as the ratio between the number of moles of units of at least one saccharide salt in the form of grafted zinc and the number of moles of units of polymer(s) of the matrix, is between 1 and 50%, preferably between 5 and 30% and very preferably between 10 and 25%.

4. Product according to one of claims 2 or 3, **characterised in that** the content of zinc in the form, of at least one saccharide salt in the form of zinc grafted in the matrix, is between 0.01 and 20%, preferably between 0.05 and 10% and very preferably between 0.1 and 6% by weight with respect to the total weight of the matrix.

5. Product according to any of claims 1 to 4, **characterised in that** said at least one saccharide salt in the form of zinc is chosen from the group consisting in zinc hyaluronate, zinc gluconate, carboxymethylcellulose zinc salt, the zinc salt of a glycosaminoglycan and a mixture thereof.

6. Product according to any of claims 1 to 5, **characterised in that** the polysaccharide salts and the polysaccharides are chosen from the group consisting in hyaluronate, condroitin sulphate, cellulosic derivatives, chitosan, alginate, carrageen, xanthan and a mixture thereof.

7. Product according to any of claims 1 to 6, **characterised in that** the matrix has the form chosen from the group comprising a solution and a cross-linked gel.

8. Product according to claim 7, **characterised in that** the matrix has the form of a cross-linked gel wherein the cross-linking rate of the matrix, defined as being the ratio between the number of moles of cross-linking agent providing the bridging and the number of moles of all of the polymer chains of the matrix, is between 0.1 and 25%, preferably between 1 and 20% and very preferably between 5 and 20%.

9. Cosmetic or pharmaceutical composition, comprising a product according to any of claims 1 to 8.

10. Composition according to claim 9, **characterised in that** it further comprises at least one agent chosen from the group consisting in an antioxidant agent, another healing agent, another anti-inflammatory agent and a mixture thereof, as a combination product for a simultaneous use, separate use and/or use spread out over time.

11. Cosmetic use of a product according to any of claims 1 to 8
wherein the content of zinc in the form of at least one saccharide salt in the form of zinc dispersed in the matrix, is between 0.01 and 10%, preferably between 0.05 and 5% and very preferably between 0.1 and 3% by weight with respect to the total weight of the matrix;
as a cosmetic agent for filling or increasing the volume of a biological tissue or for replacing or supplementing a biological fluid, in a cosmetic composition, said cosmetic composition being intended to:
(i) prevent and/or combat the signs of skin ageing; and/or
(ii) improve the texture of the skin; and/or
(iii) revive the radiance of the skin; and/or
(iv) erase and treat fine lines and/or wrinkles of the skin; and/or
(v) increase the volumes of the face or of the body; and/or
(vi) redefine the contours of the face or of the body.

12. Method of cosmetic treatment intended to
(i) prevent and/or combat the signs of skin ageing; and/or
(ii) improve the texture of the skin; and/or
(iii) revive the radiance of the skin; and/or
(iv) erase and treat fine lines and/or wrinkles of the skin; and/or
(v) increase the volumes of the face or of the body; and/or
(vi) redefine the contours of the face or of the body;
comprising the injection in the zones in question of a product such as defined according to claim 11 or of a cosmetic composition according to claim 9.

13. Product according to any of claims 1 to 8, for use as a drug having a local anti-inflammatory and healing activity, intended to:
(i) fill or replace a biological tissue; and/or
(ii) increase the volume of said biological tissue; and/or
(iii) supplement or replace a biological fluid.

14. Product according to any of claims 1 to 8, for use as a drug having a local anti-inflammatory and healing activity, intended for preventing and/or treating pathologies that can be improved or avoided by:
(i) the filling or the replacing of a biological tissue; and/or
(ii) the increasing of the volume of said biological tissue; and/or
(iii) the supplementing or the replacing of a biological fluid.

15. Product for its use according to claim 14, **characterised in that** the pathologies are chosen from the group consisting of joint degenerations such as osteoarthritis, arthritis, periodontal diseases, neurological pathologies, orthopaedic pathologies.

16. Product according to any of claims 1 to 8, for use as a drug having a local anti-inflammatory and healing activity, intended for the prevention and/or treatment of pathologies or of disorders linked to ageing or consecutive to an act of surgery.

17. Agent for filling or replacing or increasing the volume of a biological tissue, having a local anti-inflammatory and healing activity, comprising a product according to any of claims 1 to 8.

18. Agent for replacing or supplementing a biological fluid, having a local anti-inflammatory and healing activity, comprising a product according to any of claims 1 to 8.
